# EUROPEAN PATENT SPECIFICATION

(11) **EP 4 440 879 B1**
(45) Date of publication and mention of the grant of the patent: **29.01.2025**
(21) Application number: 22726383.7
(22) Date of filing: 16.02.2022
(51) Int. Cl.: B67D 9/00, H04L 67/125, G01F 15/063, G01F 15/06

(54) **INTELLIGENT SYSTEM FOR REAL-TIME MEASUREMENTS AND ANALYSIS OF FUEL OILS, FOR QUANTITATIVE AND QUALITATIVE ASSESSMENT AND ACCEPTANCE**
INTELLIGENTES SYSTEM FÜR ECHTZEITMESSUNGEN UND ANALYSE VON BRENNSTOFFÖLEN ZUR QUANTITATIVEN UND QUALITATIVEN BEURTEILUNG UND AKZEPTANZ
SYSTÈME INTELLIGENT POUR DES MESURES ET UNE ANALYSE EN TEMPS RÉEL D'HUILES COMBUSTIBLES, POUR UNE ÉVALUATION ET UNE ACCEPTATION QUANTITATIVES ET QUALITATIVES

(43) Date of publication of application: 09.10.2024
(73) Proprietor: ENSOMATOSYS GROUP LTD., 3020 Limassol (CY); Chasampalis, Dimitrios, 3106 Limassol (CY); Kontaxis, Dimitrios, 11745 Neos Kosmos Athens (GR); Vavourakis, Ioannis, 11251 Athens (GR)
(72) Inventor: CHASAMPALIS, Dimitrios, 3106 Limassol (CY); KONTAXIS, Dimitrios, 11745 Neos Kosmos, Athens (GR); VAVOURAKIS, Ioannis, 11251 Athens (GR)
(74) Representative: Samouilidis, Emmanouil
(86) International application number: PCT/EP2022/025052
(87) International publication number: WO 2023/155966

(56) References cited:
- WO-A1-2021/002803
- US-A1- 2016 133 065
- US-A1- 2017 287 237
- US-A1- 2019 324 439

## Description

### 1. Field of application

a. The Invention is mainly applied to the refueling process of ships however, potentially, it can be applied to the refueling process of all means of transport. The international term for the refueling process of ships is "bunkering". Moreover, the Invention can be applied to the loading or unloading process of fuel oils of any type, used a cargo. The main objectives of the Invention are:
   (1) Delivery - acceptance of the correct quantity of fuel.
   (2) Delivery - acceptance of fuel quality which complies with the ISO 8217: 2017 specifications or any international/national standard in force related to the refueling process or fuel cargo loading - unloading process, thus eliminating or minimizing the cases of adulterated fuel or fuel of bad quality, which may have catastrophic consequences for the ship's/mean's engines and/or the environment.
   (3) Optimal management of the refueling/bunkering or fuel cargo loading - unloading process and minimization (or even elimination) of the economic losses.
b. The Invention is a System which achieves the abovementioned objectives by collecting, monitoring, presenting and analyzing in real time numerous parameters of interest, during refueling/bunkering or fuel cargo loading - unloading process, by exploiting at least the following sub-systems:
   (1) The Portable Device.
   (2) The Cloud Application and Data Base (DB).
   (3) The Mobile Application (for smartphones or any portable device with short range wireless communication capability).

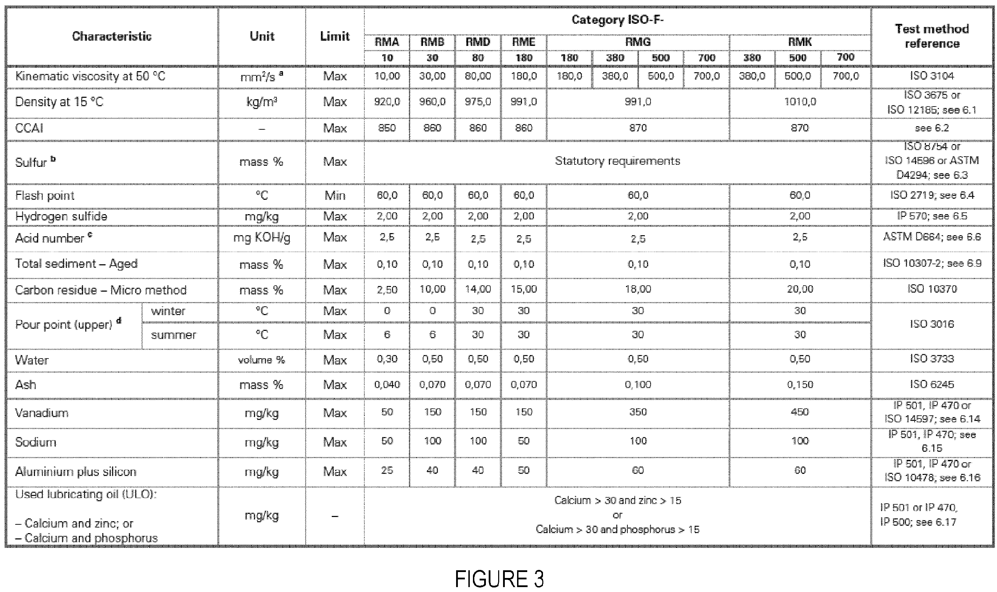

WO 2021/002803 shows a model of a skid 300 for testing and validating the method disclosed. The training skid of Figure 3(a) of WO 2021/002803 is a scaled-down version of the skid 300 (i.e. a) can be used to understand relationships and dependencies and to obtain data to train the classification model. A full size version of the skid 300, i.e. a detection skid, can be used in practice to install onboard the vessels, according to the pipeline configuration of the vessel. The instrumentation on the detection skid includes a differential pressure transducer, temperature transducers and a static pressure transducer, which are mounted onto the tapping points on the pipeline. The detection skid has a Coriolis mass flow meter.

US 2016/133065 is related to a system to assist an operator to control a vehicle in a fuel-efficient manner. The document describes a user interface presenting a real-time target to follow to maximize fuel economy and safety, achieved by modulating the accelerator pedal appropriately. This target is derived via a set of one or more algorithms that use data from the following one or more input sources, comprising the Engine Control Unit (ECU). The data includes the fuel rate.

US 2017/287237 describes a system aiming s to inform the driver of a car of the category of the fuel in a station.

US 2019/324439 discloses methods and systems for data collection in industrial environments. The systems are for use in data collection of ventilation systems, drivetrains for a mining vehicle, conveyors, fans, mining explosive system.

### 2. Description of the problem and the necessity of the Invention

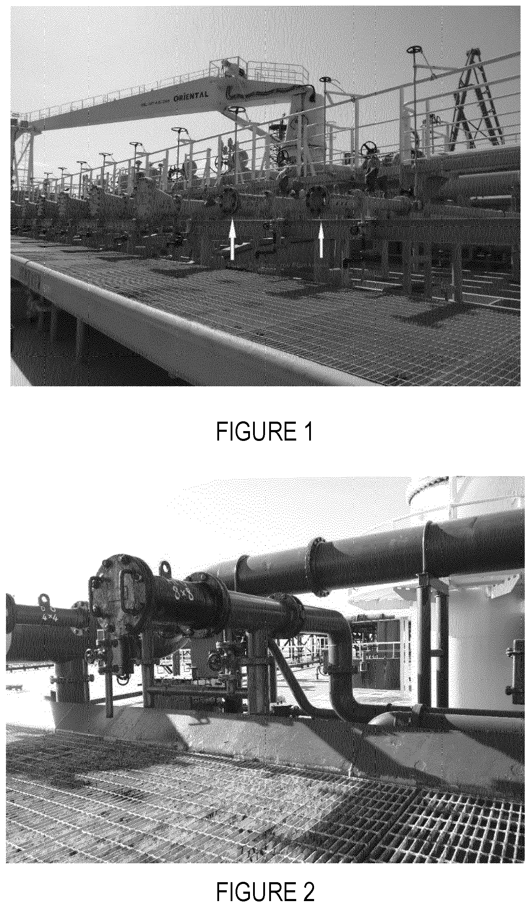

The process described below is focused but not limited to the bunkering procedures (i.e. it can be generalized to the fuel cargo loading - unloading process, refueling process of all means of transport, etc.).
a. The bunkering process of a ship involves the following steps - procedures:
   (1) Step 1: A floating fuel tank (vessel) approaches the ship to be refueled. The term used internationally for this tank is "barge".
   (2) Step 2: The barge connects its pipe to the ship's refueling pipe. The connection can be made either directly, if the cross section of the two pipes is the same, or through a "cross section converter" (referred to in shipping as "reducer"), if the cross section of the two pipes is different. The connection is made with flanges and bolts, as shown in Figures 1 and 2.
   (3) Step 3: A fuel sample collection assembly is fit/installed at the connection point between the aforementioned pipes (see Step 2). At the end of the bunkering process, the collected fuel is divided into four (4) equal samples/parts, as follows:
      (a) Two parts remain on board.
      (b) One part is delivered to the barge.
      (c) One part is sent by the ship-owning company to a third-party laboratory, which carries out a chemical analysis.
   (4) Step 4: The bunkering process starts with specific pressure (measured in bars) and fuel flow rate (measured in m³/h). The fuel temperature during bunkering must allow for smooth fuel flow. Therefore, fuels of high viscosity (measured in centistokes (cSt)) shall be supplied (from the barge) at a relatively high temperature (e.g. 40°C).
   (5) Step 5: The bunkering process ends and quantitative acceptance of the fuel follows: the ship's engineers, measure (manually) the level/ height of the fuel in the ship's tank, which corresponds to a specific volume of fuel (based on the knowledge of the tanks dimensions). This procedure is followed in all cases.
b. The qualitative acceptance of the fuel is notified after several days, after the completion of the chemical analysis by the laboratory (see Step 3 / c, above).
c. The main problems of the above process are the following:
   (1) Regarding the quantitative acceptance:
      (a) The procedure of measuring the "height" of fuel inside the ship's tank **is not commonly accepted.** The barge usually expresses objections for the accuracy of the measurement performed at the ship side, while its measurements (made with "positive displacement" or "coriolis" flowmeters) are considered, according to the international practice, the most valid ones. In these cases, which tend to be the rule, the solution is given as follows:
         1/ either by mutual compromise, **leading one of the two parties (the ship or the barge) to significant economic losses,**
         2/ or at the court, which results in court decisions which **lead again one of the two parties to significant economic losses.**
      (b) In case of **adulteration of the supplied fuel with air bubbles (from the barge),** the "height" of the fuel inside the tank of the ship is "temporarily" correct. After some period of time (and after the barge has departed and the quantitative acceptance of the fuel has taken place) the air bubbles escape from the fuel and hence, its level inside the tank decreases dramatically. However, the ship-owning company, after the departure of the barge, cannot prove the fraud which **leads to significant economic losses.** The adulteration of the supplied fuel with air bubbles is referred to in shipping as "cappuccino effect".
   (2) Regarding the **qualitative** acceptance, the chemical analysis of the fuel carried out by the laboratory, as described in paragraph 2a (3) (c) above, is always delayed for several days. In the meantime, the ship moves / travels with the fuel it received. In case the fuel quality is improper (e.g. high percentage of water or chemical elements out of limits, which cause problems in the operation of the engines), this will affect the operation, efficiency and service life of the ship's engines, with a major mid- / long-term economic impact on the ship-owning company. In case the supplied fuel is of very bad quality and causes **immediate and serious problems** in the operation of the engines, the ship shall interrupt its voyage and unload the supplied fuel immediately, a process with very high cost for the ship-owning company.
d. The added value of the present Invention lies at least in the following:
   (1) It is the **only known portable System** able to perform measurements and chemical analysis **in real time,** during bunkering, both **for quantitative and qualitative assessment and acceptance.**
   (2) The measurements and data are available **in real-time in a Cloud or a Mobile Application.**
   (3) To the best of the inventors' knowledge, there is **no other similar System** used for the bunkering process. **Simple flowmeters cannot be considered as "similar systems",** since:
      (a) They are not portable.
      (b) In most cases, they do not transmit data and do not provide notifications and/or alarms.
      (c) They do not perform chemical analysis.
   (4) Through the implemented **chemical analysis,** it can provide to the ship's personnel and to the ship-owning company **direct/real time information** about the quality of the received fuel and allows for an immediate decision for the **cessation of the bunkering process,** in case the fuel quality is out of the ISO 8217 specifications.
   (5) It can put in for a **commonly accepted System** that will minimize or eliminate:
      (a) Phenomena of fraud.
      (b) Economic losses due to:
         1/ Delivery of less (than paid) amount of fuel.
         2/ Low efficiency of ship's engines.
         3/ Damage of ship's engines.
         4/ Necessity for fuel unloading.
         5/ Court costs.

### 3. Analysis of the techno-economic objectives of the Invention

As an indicative example or case study for the usefulness of the Invention, the following incident can be mentioned (without any reference to the names of the involved companies), which has been recorded in shipping and is related to the bunkering process:
In 2011, a ship received 410 tons of fuel, type IFO-180cSt, in the region of West Africa. Responsible for the delivery of the fuel were a German and a Cypriot company (intermediary suppliers) which had bought the fuel from an African supplier. After the delivery of the fuel, the ship departed, but after 72 hours of travel, problems began to appear in its engines, due to bad fuel quality (as it was proved by the chemical analysis several days later). The need for unloading the fuel from the ship's tanks was imperative. The fuel unloading process took place in a port in Sweden (the only port available for this process, due to the dimensions of the ship). This process resulted in a total economic loss of USD 350,000 for the intermediary suppliers (i.e. the German and the Cypriot company), which were never able to claim the corresponding amount from the African supplier. In addition, the ship suffered serious damages in its engines and delayed its planned voyage for 10 days, with a significant economic impact on the ship-owning company.

Apparently, if there was a system for on-site (real-time) chemical analysis of the fuel, the bunkering process would be ceased from the very first minutes, protecting both the ship-owning company and the intermediary suppliers, from significant economic losses.

Many similar incidents have been reported in shipping. In general, ship-owners' losses in each bunkering are estimated from tens of thousands of USD, mainly due to the delivery of less fuel, to hundreds of thousands of USD, due to "non-ISO 8217" fuel deliveries (which cause damage to the ship's engines), delays, court costs, etc. The losses under consideration are estimated at 4% (at least) of the cost of the supplied fuel at each bunkering.

Therefore, for a company with a fleet of only 10 vessels, with an average fuel capacity of 1000 tn IFO-380cSt per ship, the **annual economic losses** are calculated as follows: [Number of Vessels] × [Average Fuel Capacity (tn)] × [Cost / tn (USD)] × [Number of bunkering / Year] × [4% Loss] = 10 × 1000 × 650 × 10 × 4% = $ 2,600,000.00 (Note: the Cost/tn has been considered 650 USD, based on the current fuel oil cost (Feb 2022)).

Using an intelligent system for real-time quantity and quality measurements / analysis, the ship-owing company would be able to save the aforementioned amount (on an annual basis). In addition, it will be able to protect intermediary suppliers from delivering less and / or unforeseen quality of fuel, which leads them to significant economic losses and loss of their reliability.

In order to achieve the objectives of the Invention, the System meets:
▪ High reliability.
▪ Certifications for use in the marine environment.
▪ Flexible design and ease of use - handling.
▪ Affordable cost.

### 4. System Description

The System includes the following configuration items/parts/devices:

### 4.1. Portable Measuring and Chemical Analysis Device

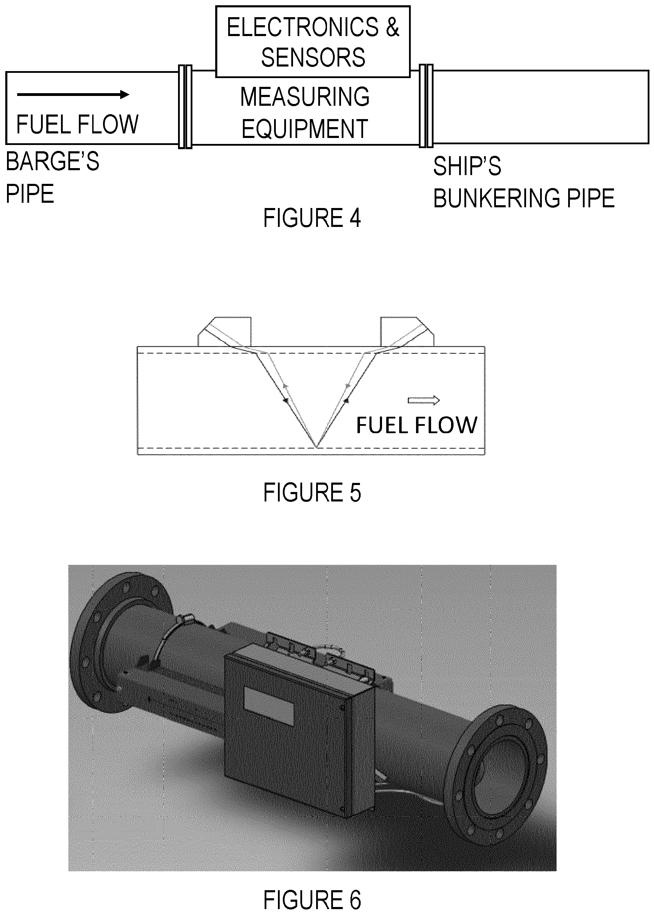

It is a portable sensor platform / device powered by a rechargeable battery. The device has the shape of a pipe and is adapted / installed at the leading edge of the ship's bunkering or fuel cargo loading-unloading pipe, in line with the fuel flow, as shown in Figure 4. The device does not interrupt or obstruct the fuel flow during the bunkering process. However, the device can also be a "fixed installation" on the ship's piping system (in line with the fuel flow) and be power supplied permanently by a DC/DC or AC/DC adapter. The device performs at least the following functions **simultaneously** and **in real time:**
a. Provides measurements of numerous parameters related to the quantitative acceptance of the fuel. Indicative parameters are listed below:
   (1) Operating and standard (normalized to any desired temperature) fuel volume rate (m³ / h).
   (2) Operating and standard (normalized to any desired temperature) fuel mass rate (tn / h).
   (3) Operating and standard (normalized to any desired temperature) total fuel volume (m³).
   (4) Operating and standard (normalized to any desired temperature) total fuel mass (tn).
b. Identifies the category/type of the fuel (e.g. RMG 380, RMG 180, Gasoil, etc.).
c. Detects possible adulteration of the fuel with air bubbles (providing alarms when the detected level exceeds a desired limit).
d. Performs instant chemical analysis of the fuel and measures:
   (1) Fuel kinematic and dynamic viscosity (cSt).
   (2) Fuel operating and standard (normalized to any desired temperature) density (kg/m³) and API degree.
   (3) Fuel temperature (°C).
   (4) Fuel humidity (% water content (H₂O)).
   (5) % content of various chemical elements in the fuel such as S, Al, Si, Ca, V, P, Zn, Na, etc. (all chemical elements of the "Periodic Table" can be detected, according to the needs of the application).
e. Detects the location of the bunkering site (WGS84 coordinates), exploiting all available Global Navigation Satellite Systems (GPS, Glonass, Beidou, Galileo).
f. Provides notifications and alarms in case any of the parameters (of paragraphs 4.1.a.-d.) exceed specific limits/thresholds, as defined in the ISO 8217: 2017 (Petroleum products - Fuels (class F) - Specifications of marine fuels) or any international/national standard in force related to the refueling or fuel cargo loading-unloading process, as shown in Figure 3. Regarding location (paragraph 4.1.e), geo-fencing notifications and alarms can be provided. These limits/thresholds can be set /configured dynamically and remotely by the System User, in the following ways:
   (1) From a Cloud Application, via the cellular network (using any available cellular technology) or satellite communications or any radio link for communication with a local router/gateway (like, but not limited to, WiFi, WiMax, LoRa, LoRaWAN, Zigbee, Bluetooth, Z-Wave, Sigfox, etc.).
   (2) From a Mobile Application, via a short-range radio link (using any short-range radio link available on Mobile Phones).
g. Implements the measurements of paragraphs 4.1.a.-e. with a remotely configurable sampling rate and transmits all bunkering/fuel cargo data:
   (1) To a Cloud Application, via the cellular network (using any available cellular technology) or satellite communications or any radio link for communication with a local router/gateway (like, but not limited to, WiFi, WiMax, LoRa, LoRaWAN, Zigbee, Bluetooth, Z-Wave, Sigfox, etc.).
   (2) To a Mobile Application, via a short-range radio link (using any short-range radio link available on Mobile Phones).
i. Stores locally all the above mentioned data.

For the implementation of the functions of paragraphs 4.1.a.-c the Portable Device employs ultrasound (u/s) technology, based on a commercial solution, specially configured and customized for the needs of the Invention. More specifically, an audio source generates and transmits periodic sound signals - pulses of appropriate frequency (Hz) which pass through the fuel and are received by a u/s receiver. This is done in the direction of the fuel flow, as well as in the opposite direction, as shown in Figure 5. The system can measure the propagation delay of the sound signals between the transmitter and the receiver, which is affected by the flow rate, the density (or the API degree) and the temperature of the fuel. Neither the u/s transmitter nor the receiver are in contact with the fuel, as shown in Figure 5. The measured propagation delay of the sound signals corresponds to a specific combination of density (or API degree) and fuel speed. Temperature measurements are also taken into account and are performed using specialized temperature probes. Knowing the density, the speed and the temperature of the fuel, as well as the dimensions of the Portable Device, the device calculates the parameters of paragraphs 4.1.a.-b., as well as the presence of air bubbles in the fuel (as described in 4.1.c), which "distort" the fuel flow and affect various diagnostic parameters monitored (in real time) by the processing unit of the device.

For the measurements of paragraphs 4.1.d.-e., the Portable Device employs various sensors of appropriate technology. All device functions, such as
▪ real time execution of measurements,
▪ real time analysis, processing and storage of measurements,
▪ real time transmission of measurements, notifications and alarms,
▪ remote configuration and Firmware (FW) updates,
are implemented by a suitable ECU (Electronic Control Unit) which includes:
▪ an embedded processing unit, based on a microprocessor unit and various interfaces,
▪ a cellular transceiver (using any available cellular technology) or a satellite transceiver or any radio link for communication with a local router/gateway (like, but not limited to, WiFi, WiMax, LoRa, LoRaWAN, Zigbee, Bluetooth, Z-Wave, Sigfox, etc.).
▪ a real time clock (RTC).

An indicative representation of the Portable Device is depicted in Figure 6.

### 4.2. Cloud Application and Data Base

The Cloud Application and the System's Data Base have been developed in order to:
a. Support all of the functions/features of the Portable Device, as described in detail in paragraph 4.1.
b. Support other functions, as follows:
   (1) Storage and processing of historic data.
   (2) Statistical and economic analysis.
   (3) User management.

### 4.3. Mobile Application

The Mobile Application has been developed in order to support the functions of paragraph 4.1. This Application shall be used on a Mobile Phone in the vicinity of the Portable Device. The Radio Access Technology (RAT) between the Mobile Phone and the Portable Device can be any short-range radio link available on Mobile Phones.

### 5. Conclusions

The Invention has been designed and a functional prototype has been put under test in certified labs. Results show that the Invention contributes to the following:
a. Delivery / acceptance of the correct **quantity** of fuel (with tolerance ≤ 0.5%).
b. Delivery - acceptance of fuel **quality** which complies with the ISO 8217: 2017 specifications, or any international/national standard in force.
c. Optimal management of the refueling/fuel cargo loading-unloading process and minimization (or even elimination) of the economic losses.

The innovation of the Invention lies in the following:
a. It is the only **portable System,** which can provide to the ship's personnel and the ship-owning company **real time** information about the **quantity** & **quality** of the received fuel, enabling an early **shutdown / cessation** of the bunkering/fuel cargo loading-unloading process, in case certain conditions and limits/thresholds are not met. In this context, the Invention could put in for a commonly accepted System, able to minimize or eliminate economic losses and phenomena of fraud.
b. Any refueling/fuel cargo loading-unloading process is supported by a Cloud Application and Data Base, as well as by a Mobile Application, which allow for:
   ▪ Remote real-time data acquisition.
   ▪ Remote real-time notifications and alarms.
   ▪ Remote management, configuration and Firmware updates of the Portable Device.
   ▪ Data analytics.
   ▪ Preservation of historic data and statistics.

## Claims

1. System for real-time measurements and analysis of fuel oils and the quantitative and qualitative assessment and acceptance of the fuel, which is configured to perform the acquisition, monitoring, presentation, analysis and storage of numerous parameters in **real time,** during a ship's refueling or fuel cargo loading-unloading process, and is implemented using: a cloud application, a mobile application, and a portable device, **characterized in that** the portable device is configured to be installed at the leading edge of the ship's bunkering or fuel cargo loading-unloading pipe, in line with the fuel flow and including i) means employing ultrasound technology and ii) sensors to perform chemical analysis both for quantitative and qualitative assessment and acceptance, i.e. measuring the % content of a plurality of chemical elements in the fuel.

2. System according to claim 1, whereby the means employing ultrasound technology are means to i) provide measurements of parameters related to the quantity of the fuel, ii) identify the category/type of the fuel and iii) detect adulteration of the fuel with air bubbles.

3. System according to claim 1 or claim 2, whereby the portable device has the shape of a pipe.

4. System according to anyone of claims 1 to 3, whereby the portable device includes an Electronic Control Unit (abb.: ECU) including an embedded processing unit, a cellular transceiver or a satellite transceiver or a radio link for communication with a local router/gateway and a real time clock, which ECU is connected with sensors and is configured to transmit measurements stored in the ECU to the cloud application and the mobile application.

5. Method to use a system according to anyone of claims 1 to 4, including:
i. installing the portable device at the leading edge of the ship's bunkering or fuel cargo loading-unloading pipe, in line with the fuel flow
ii. measuring parameters related to the quantity of the fuel
iii. identifying the category/type of the fuel
iv. detecting adulteration of the fuel with air bubbles if any and providing alarms when the detected level exceeds a desired limit)
v. performing instant chemical analysis of the fuel
vi. setting threshold limits of any of said parameters, category/type of fuel, adulteration of the fuel with air bubbles, chemical analysis
vii. providing notifications and alarms in case any threshold limit is exceeded.

6. Method according to claim 5, including setting threshold limits of any of said parameters, category/type of fuel, adulteration of the fuel with air bubbles, chemical analysis as defined in ISO 8217:2017.

7. Method according to claim 5 or claim 6, including identifying the category/type of the fuel according to ISO 8217:2017.

8. Device for real-time measurements and analysis of fuel oils and the quantitative and qualitative assessment and acceptance of the fuel that is configured to perform the acquisition, monitoring, presentation, analysis and storage of numerous parameters in real time, during a ship's refueling or fuel cargo loading-unloading process, **characterized in that:**
the device is a portable device configured to be installed at the leading edge of the ship's bunkering or fuel cargo loading-unloading pipe, in line with the fuel flow and including means employing ultrasound technology and sensors to perform chemical analysis, i.e. measuring the % content of a plurality of chemical elements in the fuel, and
the device has an Electronic Control Unit (abb.: ECU) including an embedded processing unit, a cellular transceiver or a satellite transceiver or a radio link for communication with a local router/gateway and a real time clock, which ECU is connected with sensors and is configured to transmit measurements stored in the ECU to a cloud application and a mobile application.

## Patentansprüche

1. System zur Echtzeitmessung und -analyse von Heizölen und zur quantitativen und qualitativen Bewertung und Abnahme des Brennstoffs, das so konfiguriert ist, um die Erfassung, Überwachung, Darstellung, Analyse und Speicherung zahlreicher Parameter **in Echtzeit** während eines Betankungsprozesses eines Schiffes oder eines Be- und Entladens einer Brennstoffladung durchzuführen, und das unter Verwendung einer Cloud-Anwendung, einer mobilen Anwendung und einer tragbaren Vorrichtung implementiert ist, **dadurch gekennzeichnet, dass**
die tragbare Vorrichtung so konfiguriert ist, dass sie an der Vorderkante des Bunker- oder Brennstoffladung-Lade-/Entladerohrs des Schiffes in einer Linie mit dem Brennstofffluss installiert wird und i) Mittel, die Ultraschalltechnologie einsetzen, und ii) Sensoren zum Durchführen chemischer Analysen sowohl zur quantitativen als auch zur qualitativen Bewertung und Abnahme, d. h. zur Messung des prozentualen Gehalts einer Vielzahl chemischer Elemente im Brennstoff, beinhaltet.

2. System nach Anspruch 1, wobei die Mittel, die Ultraschalltechnologie einsetzen, Mittel sind, um I) Messungen von Parametern bereitzustellen, die mit der Menge des Brennstoffs zusammenhängen, ii) die Kategorie/den Typ des Brennstoffs zu identifizieren und iii) eine Verfälschung des Brennstoffs mit Luftblasen zu erkennen.

3. System nach Anspruch 1 oder Anspruch 2, wobei die tragbare Vorrichtung die Form eines Rohrs aufweist.

4. System nach einem der Ansprüche 1 bis 3, wobei die tragbare Vorrichtung eine elektronische Steuereinheit (kurz: ECU) beinhaltet, die eine eingebettete Verarbeitungseinheit, einen Mobilfunk-Transceiver oder einen Satelliten-Transceiver oder eine Funkverbindung zur Kommunikation mit einem lokalen Router/Gateway und eine Echtzeituhr beinhaltet, wobei die ECU mit Sensoren verbunden und so konfiguriert ist, dass sie in der ECU gespeicherte Messungen an die Cloud-Anwendung und die mobile Anwendung überträgt.

5. Verfahren zur Verwendung eines Systems nach einem der Ansprüche 1 bis 4, das Folgendes beinhaltet:
i. Installieren der tragbaren Vorrichtung an der vorderen Kante des Bunker-oder Brennstoffladung-Lade-/Entladerohrs des Schiffes, in einer Linie mit dem Brennstofffluss
ii. Messen von Parametern, die sich auf die Menge des Brennstoffs beziehen
iii. Identifizieren der Kategorie/des Typs des Brennstoffs
iv. Erkennen einer eventuellen Verfälschung des Brennstoffs durch Luftblasen und Auslösen von Alarmen, wenn der ermittelte Wert einen gewünschten Grenzwert überschreitet
v. Durchführen einer sofortigen chemischen Analyse des Brennstoffs
vi. Festlegen von Grenzwerten für jeden der Parameter, die Kategorie/Typ des Brennstoffs, die Verfälschung des Brennstoffs durch Luftblasen, die chemische Analyse
vii. Bereitstellen von Benachrichtigungen und Alarmen bei Überschreitung eines Schwellenwerts.

6. Verfahren nach Anspruch 5, das Festlegen von Grenzwerten für einen der Parameter, die Kategorie/den Typ des Brennstoffs, die Verfälschung des Brennstoffs mit Luftblasen und die chemische Analyse gemäß ISO 8217:2017 beinhaltet.

7. Verfahren nach Anspruch 5 oder Anspruch 6, das Identifizieren der Kategorie/des Typs des Brennstoffs gemäß ISO 8217:2017 beinhaltet.

8. Vorrichtung zur Echtzeitmessung und -analyse von Heizölen und zur quantitativen und qualitativen Bewertung und Abnahme des Brennstoffs, die so konfiguriert ist, die Erfassung, Überwachung, Darstellung, Analyse und Speicherung zahlreicher Parameter in Echtzeit während eines Betankungsprozesses eines Schiffes oder eines Be- und Entladens einer Brennstoffladung durchzuführen, **dadurch gekennzeichnet, dass**
es sich bei der Vorrichtung um eine tragbare Vorrichtung handelt, die so konfiguriert ist, an der Vorderkante des Bunker- oder Brennstoffladung-Lade-/Entladerohrs des Schiffes in einer Linie mit dem Brennstofffluss installiert zu werden und Mittel, die Ultraschalltechnologie einsetzen, und Sensoren zum Durchführen chemischer Analysen, d. h. zum Messen des prozentualen Gehalts einer Vielzahl chemischer Elemente im Brennstoff, beinhaltet und
die Vorrichtung eine elektronische Steuereinheit (kurz: ECU) aufweist, die eine eingebettete Verarbeitungseinheit, einen Mobilfunk-Transceiver oder einen Satelliten-Transceiver oder eine Funkverbindung zur Kommunikation mit einem lokalen Router/Gateway und eine Echtzeituhr beinhaltet, wobei die ECU mit Sensoren verbunden und so konfiguriert ist, dass sie in der ECU gespeicherte Messungen an eine Cloud-Anwendung und eine mobile Anwendung überträgt.

## Revendications

1. Système pour des mesures et analyses en temps réel de fiouls et l'évaluation et l'acceptation quantitatives et qualitatives du combustible, qui est configuré pour effectuer l'acquisition, la surveillance, la présentation, l'analyse et le stockage de nombreux paramètres en **temps réel,** pendant le processus de ravitaillement en combustible ou de chargement-déchargement de cargaison de combustible d'un navire, et est mis en oeuvre en utilisant : une application cloud, une application mobile et un dispositif portable, **caractérisé en ce que**
le dispositif portable est configuré pour être installé au bord d'attaque du tuyau de chargement-déchargement de cargaison de combustible ou de soutage du navire, en ligne avec le flux de combustible et incluant i) des moyens employant la technologie des ultrasons et ii) des capteurs pour effectuer une analyse chimique à la fois pour l'évaluation et l'acceptation quantitatives et qualitatives, c'est-à-dire la mesure de la teneur en % d'une pluralité d'éléments chimiques dans le combustible.

2. Système selon la revendication 1, selon lequel les moyens employant la technologie des ultrasons sont des moyens pour I) fournir des mesures de paramètres liés à la quantité de combustible, ii) identifier la catégorie/le type du combustible et III) détecter l'adultération du combustible avec des bulles d'air.

3. Système selon la revendication 1 ou la revendication 2, selon lequel le dispositif portable présente la forme d'un tuyau.

4. Système selon l'une quelconque des revendications 1 à 3, selon lequel le dispositif portable inclut une Unité de Commande Électronique (abb. : ECU) incluant une unité de traitement intégrée, un émetteur-récepteur cellulaire ou un émetteur-récepteur satellite ou une liaison radio pour la communication avec un routeur/une passerelle local(e) et une horloge en temps réel, laquelle ECU est connectée à des capteurs et est configurée pour transmettre les mesures stockées dans l'ECU à l'application cloud et à l'application mobile.

5. Procédé d'utilisation d'un système selon l'une quelconque des revendications 1 à 4, incluant :
i. l'installation du dispositif portable au niveau du bord d'attaque du tuyau de chargement-déchargement de cargaison de combustible ou de soutage du navire, en ligne avec le flux de combustible
ii. la mesure de paramètres liés à la quantité de combustible
iii. l'identification de la catégorie/du type du combustible
iv. la détection de l'adultération du combustible avec des bulles d'air, le cas échéant, et la fourniture d'alarmes lorsque le niveau détecté dépasse une limite souhaitée
v. la réalisation d'une analyse chimique instantanée du combustible
vi. la définition de limites de seuil de l'un quelconque desdits paramètres, catégorie/type de combustible, adultération du combustible par des bulles d'air, analyse chimique
vii. la fourniture de notifications et d'alarmes en cas de dépassement de toute limite de seuil.

6. Procédé selon la revendication 5, incluant la définition de limites de seuil de l'un quelconque desdits paramètres, catégorie/type de combustible, adultération du combustible par des bulles d'air, analyse chimique tels que définis dans la norme ISO 8217:2017.

7. Procédé selon la revendication 5 ou la revendication 6, incluant l'identification de la catégorie/du type du combustible selon la norme ISO 8217:2017.

8. Dispositif pour des mesures et analyses en temps réel de fiouls et l'évaluation et l'acceptation quantitatives et qualitatives du combustible qui est configuré pour effectuer l'acquisition, la surveillance, la présentation, l'analyse et le stockage de nombreux paramètres en temps réel, pendant le processus de ravitaillement en combustible ou de chargement-déchargement de cargaison de combustible d'un navire, **caractérisé en ce que** :
le dispositif est un dispositif portable configuré pour être installé au niveau du bord d'attaque du tuyau de chargement-déchargement de cargaison de combustible ou de soutage du navire, en ligne avec le flux de combustible et incluant des moyens employant la technologie des ultrasons et des capteurs pour effectuer une analyse chimique, c'est-à-dire la mesure de la teneur en % d'une pluralité d'éléments chimiques dans le combustible, et
le dispositif présente une Unité de Commande Électronique (abb. : ECU) incluant une unité de traitement intégrée, un émetteur-récepteur cellulaire ou un émetteur-récepteur satellite ou une liaison radio pour la communication avec un routeur/une passerelle local(e) et une horloge en temps réel, laquelle ECU est connectée à des capteurs et est configurée pour transmettre les mesures stockées dans l'ECU à une application cloud et à une application mobile.
